Europäisches Patentamt

European Patent Office

Office européen des brevets

(19)

(11) Numéro de publication : **0 332 525 B1**

(12) **FASCICULE DE BREVET EUROPEEN**

(45) Date de publication du fascicule du brevet :
**08.04.92 Bulletin 92/15**

(51) Int. Cl.$^5$ : **C07C 43/04,** C07C 41/06,
C07C 41/42, B01D 3/14,
B01J 8/04

(21) Numéro de dépôt : **89400623.8**

(22) Date de dépôt : **06.03.89**

(54) **Procédé de préparation d'un éther alkylique tertiaire par distillation réactive.**

(30) Priorité : **08.03.88 FR 8803095**

(43) Date de publication de la demande :
**13.09.89 Bulletin 89/37**

(45) Mention de la délivrance du brevet :
**08.04.92 Bulletin 92/15**

(84) Etats contractants désignés :
**AT BE DE ES GB IT NL**

(56) Documents cités :
**EP-A- 0 008 860
GB-A- 2 096 603
US-A- 3 629 478
US-A- 4 471 154**

(73) Titulaire : **INSTITUT FRANCAIS DU PETROLE
4, Avenue de Bois Préau
F-92506 Rueil-Malmaison Cédex (FR)**
Titulaire : **ELF FRANCE, Société Anonyme
dite:
Tour Elf 2 place de la Coupole La Défense 6
F-92400 Courbevoie (FR)**

(72) Inventeur : **Nocca, Jean-Luc
7, Boulevard Marcel Pourtout
F-92500 Rueil Malmaison (FR)**
Inventeur : **Leonard, Jacques
15, rue Anatole France
F-95370 Montigny (FR)**
Inventeur : **Gaillard, Jean-Ferdinand
rue du Commandant Charcot
F-69000 Lyon (FR)**
Inventeur : **Amigues, Pierre
rue Felin
F-69340 Francheville (FR)**

EP 0 332 525 B1

Jouve, 18, rue Saint-Denis, 75001 PARIS

**Description**

La présente invention est relative à un procédé de préparation d'un éther alkylique tertiaire par réaction d'un alcool aliphatique avec un mélange d'hydrocarbures renfermant au moins une iso-olefine.

Elle est également relative à un appareil dans lequel un tel procédé peut être mis en oeuvre.

La présente invention concerne plus particulièrement la préparation du méthyl tertio-butyl éther (MTBE) à partir d'isobutène et de méthanol, la préparation du méthyl tertio-amyl éther (TAME) à partir d'isopentène et de méthanol et la préparation de MTBE et de TAME à partir d'isobutène, d'isopentène et de méthanol.

Les éthers alkyliques tertiaires, en particulier le méthyl tertio-butyl éther (MTBE) et le méthyl tertio-amyl éther (TAME), constituent des additifs très intéressants pour l'amélioration des qualités des essences.

Ainsi, le méthyl tertio-butyl éther (MTBE) permet, en raison de ses propriétés antidétonantes, d'améliorer la qualité des essences commerciales, avec obtention d'un indice d'octane plus élevé que celui obtenu par l'addition de méthanol, l'un des additifs les plus intéressants. En outre, le méthyl tertio-butyl éther (MTBE) possède un pouvoir calorifique plus élevé que celui du méthanol : 8395 kcal/kg (soit 35 091 kJoule/kg) pour le MTBE, contre 4764 kcal/kg (soit 19 914 kJoule/kg) pour le méthanol (en moyenne, le pouvoir calorifique d'un super carburant est 10 200 kcal/kg, soit 42 636 kJoule/kg). De plus, le MTBE ne donne pas lieu à des problèmes de démixion en présence d'eau, comme c'est le cas pour le méthanol. Enfin, la solubilité de l'eau dans le MTBE étant considérablement supérieure a celle de l'eau dans les hydrocarbures, il s'ensuit que l'ajout de MTBE améliore la tolérance à l'eau des carburants.

Il est connu de préparer des éthers alkyliques tertiaires, en particulier le méthyl tertio-butyl éther (MTBE) et le méthyl tertio-amyl éther (TAME) qui sont parmi les éthers les plus courants, en faisant réagir une iso-oléfine généralement contenue dans une fraction d'hydrocarbures avec un alcool, par exemple le méthanol, en présence d'un catalyseur acide, par exemple l'acide sulfurique, l'acide fluorhydrique, le chlorure d'aluminium ou le fluorure de bore, ou en présence de matières carbonées contenant des groupes - $SO_3H$, par exemple des charbons sulfonés, des résines phénol-formaldéhyde sulfonées, des polymères coumarone-indène sulfonés ou, de préférence, des résines polystyrène-divinylbenzène sulfonées.

On sait depuis longtemps que la réaction entre le méthanol et les oléfines tertiaires est équilibrée et qu'il est donc difficile d'obtenir des taux de conversion élevés. Par ailleurs, l'équilibre est d'autant moins déplacé vers la formation d'éthers que le poids moléculaire de l'iso-oléfine est plus élevé. Ainsi, dans le cas des iso-amylènes, les taux de conversion restent limités à 65 - 75 % si l'on ne désire pas mettre en oeuvre des excès de méthanol trop importants. Si l'on désire obtenir des taux acceptables de conversion des iso-oléfines, en particulier des iso-amylènes, il faut mettre en oeuvre des excès de méthanol très élevés par rapport à la stoechiométrie ; mais on se retrouve alors avec un effluent réactionnel contenant des quantités de méthanol trop importantes pour pouvoir être enlevées facilement par des voies classiques telles que la distillation azéotropique avec des hydrocarbures et le recyclage au réacteur, tel que décrit dans le brevet FR-B2411881. D'après le brevet US-A 4204077, le méthanol pourrait être alors éliminé par une extraction avec un solvant tel que l'éthylène glycol.

Dans ces techniques traditionnelles, l'éther alkylique tertiaire, qui s'est formé par réaction d'un alcool avec une iso-oléfine contenue dans un mélange d'hydrocarbures, se retrouve au sein d'un mélange d'hydrocarbures et parfois d'alcool non convertis. Aussi, une fois faite cette réaction d'éthérification dans, en général, au moins deux réacteurs, il faut séparer l'éther alkylique tertiaire des autres constituants dans plusieurs colonnes de distillation tout en éliminant le maximum d'alcool avec le minimum de pertes en éther.

Ainsi, l'utilisation de plusieurs réacteurs et colonnes de distillation pour préparer et séparer l'éther alkylique tertiaire augmente les coûts d'investissement et de fonctionnement tout en ne garantissant pas un excellent rendement en éther.

Il a été proposé une méthode pour résoudre les problèmes en question : c'est la méthode de la distillation réactive (ou distillation catalytique), qui consiste à effectuer dans la même enceinte la réaction d'éthérification avec un catalyseur et la distillation pour séparer l'éther alkylique tertiaire des autres constituants non convertis au fur et à mesure qu'il se forme (US-A 3629 478, EP-B 0008 860, FR-B 2503700).

Dans le brevet US-A 3629 478, le catalyseur est disposé en vrac dans les goulottes de déversement (ou descentes) des plateaux perforés de distillation : selon ce brevet, seule la phase liquide descendante est en contact avec le catalyseur, la phase vapeur montant à travers les perforations de chaque plateau de distillation. En fait, en raison du caractère exothermique de la réaction, une phase vapeur se forme inévitablement au contact du catalyseur, d'où un problème d'hydrodynamique : il sera en effet très difficile, si ce n'est impossible, pour une phase mixte constituée par le liquide plus de la vapeur formée par la chaleur de réaction, c'est-à-dire une phase légère, de descendre dans les goulottes de déversement, car le catalyseur contenu dans celles-ci offre une grande résistance à son passage du fait de la faible section desdites goulottes, donc de la faible section catalytique.

Le brevet EP-B 0008 860 propose d'alimenter, avec un mélange contenant de l'isobutène et du méthanol, une colonne de distillation remplie d'un catalyseur approprié pour la préparation du méthyl tertio-butyl éther (MTBE), dans laquelle le catalyseur agit également comme garnissage pour la distillation, formant ainsi le MTBE et le séparant en même temps des constituants à quatre atomes de carbone.

Bien que le procédé décrit dans ce brevet représente un apport technique important dans le domaine de la distillation réactive, le procédé selon la présente invention permet de l'améliorer sensiblement grâce, en particulier, à une fonction de distillation plus importante.

Le brevet FR-B 2503700 propose d'utiliser une série d'étapes catalytiques avec une circulation ascendante vapeur-liquide dans chaque lit catalytique, le catalyseur étant noyé. Mais l'effet de distillation n'est pas aussi important que prévu. De plus, un problème d'hydrodynamique peut se poser : en effet, du fait de la gravité, il ne sera pas toujours aisé pour le liquide de monter à travers chaque lit catalytique.

La présente invention permet de remédier aux inconvénients mentionnés précédemment : on effectue à la fois et dans une seule et même enceinte la réaction, avec un catalyseur approprié, conduisant à la formation de l'éther alkylique tertiaire, et la séparation de ce dernier, par distillation, des hydrocarbures et des composés qui l'accompagnent, tout en ayant des rendements très satisfaisants en éther grâce, surtout, à un très bon effet de distillation.

Les figures 1,2 et 3 données à titre d'exemples, illustrent de manière non limitative l'invention ;

la figure 1 concerne le milieu de l'enceinte, la figure 2 le haut de l'enceinte, la figure 3 le bas de l'enceinte.

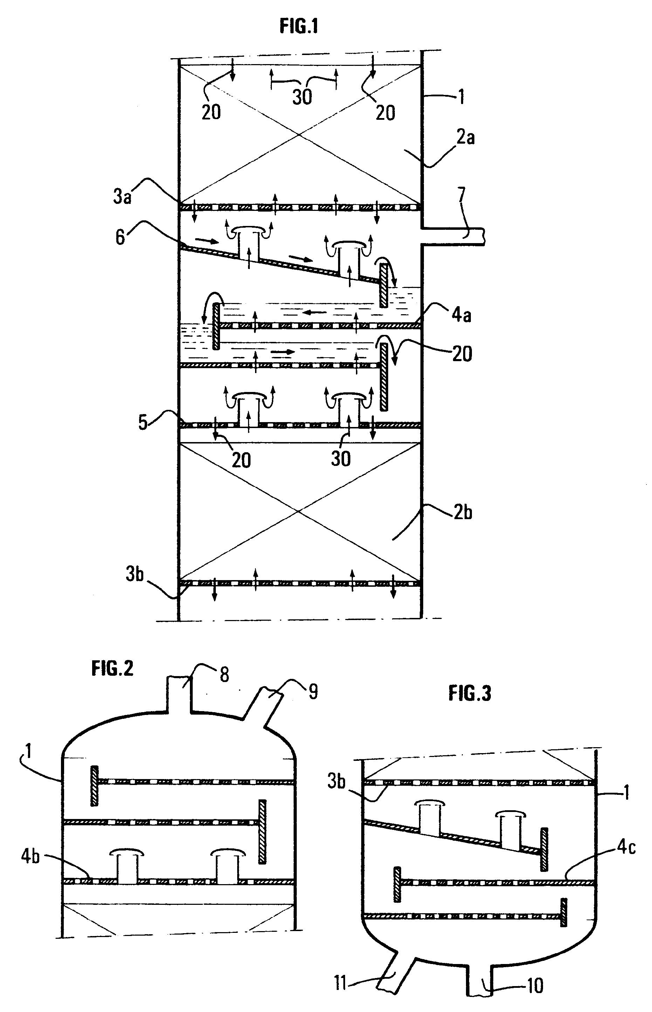

L'objet de la présente invention est un procédé de préparation d'un (ou plusieurs) éther(s) alkylique(s) tertiaire(s), par réaction d'un alcool aliphatique avec un mélange d'hydrocarbures renfermant au moins une iso-oléfine, dans une zone de réaction-distillation (c'est-à-dire une zone ou s'effectuent la réaction et/ou la distillation) définie (voir figures 1,2 et 3) par une enceinte (1) de forme sensiblement cylindrique, par exemple verticale, et contenant au moins un catalyseur du type résine sulfonée, par exemple une résine polystyrène-divinylbenzène sulfonée, ce procédé étant caractérisé :

– en ce qu'on introduit la charge de réactifs contenant au moins ledit alcool et au moins ledit mélange d'hydrocarbures dans ladite zone de réaction-distillation qui renferme :

a) au moins deux lits fixes superposés (2a,2b) et non contigus dudit catalyseur de type résine sulfonée, chacun de ces lits étant disposé sur un support ou fond perforé (3a,3b) (qui peut être, par exemple, un disque perforé, une grille support ou un filet), et comprenant une pluralité de poches de tissu renfermant ledit catalyseur, lesdites poches ménageant des espaces libres entre elles, lesdits lits de catalyseur pouvant avoir des épaisseurs différentes,

b) au moins une zone de distillation, libre de catalyseur (c'est-à-dire sans catalyseur), située dans au moins un espace libre se trouvant entre deux lits consécutifs de catalyseur (2a,2b),

c) au moins un plateau discontinu (c'est-à-dire muni d'une pluralité de discontinuités) de redistribution de liquide (5), libre de catalyseur, situé dans au moins un espace libre se trouvant entre une zone de distillation et le lit de catalyseur (ou lit catalytique) situé immédiatement en-dessous de ladite zone de distillation,

– en ce qu'on maintient des conditions de distillation dans ladite zone de réaction-distillation, de façon à avoir une phase liquide descendante et une phase vapeur ascendante dans ladite zone,

– en ce qu'on décharge au sommet (8) de la zone de réaction-distillation une phase vapeur renfermant principalement des hydrocarbures non-convertis, et

– en ce qu'on soutire à la base (10) de la zone de réaction-distillation une phase liquide renfermant principalement le(s)dit(s) éther(s) alkylique(s) tertiaire(s).

On entend par zone de distillation tout dispositif connu de l'homme de l'art pour effectuer une distillation tel que :

– au moins un garnissage inerte muni d'un support, et/ou

– au moins un plateau de distillation discontinu (4a) (c'est-à-dire muni de discontinuités pour le passage de la phase vapeur, et d'au moins une conduite de déversement (ou descente) bordée d'un déversoir pour l'écoulement de la phase liquide) pouvant être choisi notamment parmi les types suivants :

. plateaux perforés,

. plateaux à clapets,

. plateaux à calottes.

Chaque plateau de redistribution de liquide (5) est muni d'une pluralité de discontinuités. Au moins une de ses discontinuités est une cheminée ou conduite pour le passage de la phase vapeur, cheminée ou conduite dont l'extrêmité supérieure est de préférence saillante, c'est-à-dire, s'élève ou déborde au-dessus du fond dudit plateau de redistribution de liquide (5) et dont ladite extrêmité est de préférence couverte (mais non fermée), empêchant ainsi le liquide présent sur ledit plateau de se déverser dans cette cheminée ou conduite. Ses autres discontinuités sont des orifices pour le passage de la phase liquide, ces orifices étant répartis de telle manière qu'ils permettent d'arroser uniformément, avec de la phase liquide, le lit catalytique situé immédiatement au-dessous de chaque plateau de redistribution de liquide.

Dans chacun des lits catalytiques, les espaces laissés libres autour des poches de tissu contenant le catalyseur (poches qui sont perméables au liquide mais imperméables aux particules catalytiques solides) permettent le libre passage de liquide et de vapeur, donc un certain effet de distillation, celui-ci étant sensiblement amélioré par la présence de la ou des zones de distillation.

De manière avantageuse, au moins une partie de la phase vapeur déchargée au sommet (8) de la zone de réaction-distillation peut être condensée (dans un condenseur situé à l'extérieur de l'enceinte (1) définissant ladite zone et non représenté sur les figures), puis renvoyée dans ladite zone, par exemple vers le sommet de ladite zone (9), sous forme de débit liquide dénommé reflux.

De même, au moins une partie de la phase liquide soutirée à la base (10) de la zone de réaction-distillation peut être vaporisée (par passage dans un rebouilleur situé à l'extérieur de l'enceinte (1) et non représenté sur les figures) et, ensuite, réintroduite dans ladite zone, par exemple vers la base de ladite zone (11), sous forme de débit vapeur dénommé vapeur de rebouillage.

Selon un mode de réalisation préférentielle de l'invention (voir figure 1) dans le cas où au moins une zone de distillation est constituée par un ou plusieurs plateaux de distillation, la phase liquide arrive, sur chaque plateau de distillation situé immédiatement au-dessous d'un lit catalytique, le plus loin du déversoir de celui-ci, c'est-à-dire à l'opposé dudit déversoir, après être passée préalablement sur un plateau de répartition de liquide (6), libre de catalyseur, traversé par au moins une conduite ou cheminée pour le passage de la phase vapeur (conduite ou cheminée dont l'extrêmité supérieure est de préférence saillante, c'est-à-dire s'élève ou déborde au-dessus du fond du plateau, et dont ladite extrêmité peut être munie d'une calotte, empêchant ainsi le liquide de se déverser dans cette conduite ou cheminée), sensiblement incliné, situé dans chaque espace libre se trouvant entre un plateau de distillation et le lit catalytique situé immédiatement au-dessus dudit plateau de distillation, chaque plateau de répartition de liquide (6) étant en outre muni à son extrêmité la plus basse d'au moins un passage libre (qui est de préférence une goulotte ou conduite de déversement bordée d'une petite margelle) pour la phase liquide : l'écoulement du liquide dans la descente du plateau de distillation est ainsi plus régulière et la distillation sur ledit plateau de distillation encore plus efficace.

L'espace situé entre le sommet de la zone de réaction-distillation (c'est-à-dire le sommet de l'enceinte) et le lit catalytique situé le plus haut dans ladite zone (voir figure 2) peut contenir, de préférence, une zone de distillation, libre de catalyseur (par exemple, au moins un plateau de distillation discontinu (4b)).

L'espace situé entre la base de la zone de réaction-distillation (c'est-à-dire la base de l'enceinte) et le lit catalytique situé le plus bas dans ladite zone (voir figure 3) peut également renfermer, préférentiellement, une zone de distillation, libre de catalyseur (par exemple, au moins un plateau de distillation discontinu (4c)).

Une autre forme de réalisation préférentielle de l'invention (voir figure 1) consiste à introduire la charge de réactifs, contenant au moins l'alcool aliphatique et au moins le mélange d'hydrocarbures, dans la zone de réaction-distillation dans une position (7) telle qu'au moins un lit catalytique se trouve au-dessus de ladite position, et, de manière encore plus préférée, telle que le lit catalytique situé le plus bas dans ladite zone se trouve au-dessus de ladite position.

On peut éventuellement, en plus de la charge, introduire dudit alcool, pris isolément (c'est-à-dire seul), dans la zone de réaction-distillation au moins en un point différent du point d'introduction de ladite charge et, de préférence, situé au voisinage du sommet de cette zone de réaction-distillation (par exemple au-dessus du lit de catalyseur situé le plus haut dans ladite zone et, préférentiellement, en-dessous du point d'alimentation du reflux).

Ce complément d'alcool favorise la réaction d'éthérification et permet d'obtenir de meilleurs taux de conversion de l'iso-oléfine. De plus, il permet de réduire la formation éventuelle de dimères. La température de l'alcool injecté est, de préférence, inférieure à sa température d'ébullition.

Selon un autre mode de réalisation de l'invention, les poches de tissu renfermant le catalyseur peuvent être accrochées à des grilles enroulées sur elles-mêmes, superposées les unes sur les autres et ayant leurs mailles en majeure partie métalliques (chaque lit de catalyseur comprenant ainsi au moins une couche desdites grilles).

On maintient en général un rapport de reflux par rapport au distillat (c'est-à-dire un rapport entre le liquide reflué et le liquide soutiré) compris entre 0,1:1 et 20:1, de préférence entre 0,5:1 et 10:1. On opère le plus souvent, à l'intérieur de l'enceinte (1), dans un domaine de pression et de température relativement large : par exemple, de 1 à 30 bars (soit de 100 à 3000 kPa), de préférence de 2 à 20 bars (soit de 200 à 2000 kPa) pour la pression et de 10 à 200 °C, de préférence de 40 à 160 °C pour la température dans toute l'enceinte.

Chaque lit catalytique utilisé dans la présente invention occupe toute la section circulaire de la zone de réaction-distillation, c'est-à-dire toute la section circulaire de l'enceinte (1).

Sur la figure 1, donnée à titre d'exemple, la phase liquide suit préférentiellement le chemin indiqué par les flèches 20 et la phase vapeur suit préférentiellement le chemin indiqué par les flèches 30.

Au fur et à mesure qu'elle descend dans la zone de réaction-distillation, la phase liquide s'enrichit progressivement en éther alkylique tertiaire qui est moins volatil que l'alcool et l'iso-oléfine n'ayant pas encore réagi et que les autres constituants non éthérifiables du mélange d'hydrocarbures, qui au contraire de l'éther alkylique tertiaire ont tendance à monter vers le sommet de la zone de réaction-distillation. Ainsi la phase liquide soutirée à la base de cette zone contient principalement de l'éther alkylique tertiaire. De plus, la phase vapeur déchargée au sommet de ladite zone ne contient d'alcool non converti qu'en général sous forme de traces.

La présente invention a également pour objet (voir figures 1,2 et 3) un appareil (1), de forme sensiblement cylindrique, comportant au moins une conduite (7) pour l'introduction d'une charge, au moins une conduite (8) située au sommet dudit appareil pour le soutirage d'une phase vapeur, au moins une conduite (9) située au voisinage du sommet dudit appareil pour l'alimentation d'un reflux, au moins une conduite (10) située à la base dudit appareil pour le soutirage d'une phase liquide, au moins une conduite (11) pour l'introduction d'une phase au moins partiellement vaporisée, cet appareil étant caractérisé en ce qu'il renferme :

a) au moins deux lits catalytiques fixes, superposés et non contigus (2a,2b), chacun de ces lits étant disposé sur un support ou fond perforé (3a,3b) (qui peut être, par exemple, un disque perforé, une grille support ou un filet) et occupant toute la section circulaire interne de l'appareil (1),

b) au moins un dispositif de distillation, situé dans au moins un espace libre se trouvant entre deux lits catalytiques consécutifs (2a, 2b), et

c) au moins un plateau discontinu (c'est-à-dire muni d'une pluralité de discontinuités, comme par exemple des conduites ou cheminées dont l'extrêmité supérieure peut être saillante et couverte) de redistribution de liquide (5), situé dans au moins un espace libre se trouvant entre un dispositif de distillation et le lit catalytique situé immédiatement en-dessous dudit dispositif de distillation.

On entend par dispositif de distillation tout dispositif connu de l'homme de l'art pour effectuer une distillation tel que :

– au moins un garnissage inerte muni d'un support, et/ou

– au moins un plateau de distillation discontinu (4a) (c'est-à-dire muni de discontinuités pour le passage de la phase vapeur, et d'au moins une conduite de déversement (ou descente) bordée d'un déversoir pour l'écoulement de la phase liquide) pouvant être choisi notamment parmi les types suivants :

. plateaux perforés,

. plateaux à clapets,

. plateaux à calotte.

L'appareil (1) selon la présente invention peut comporter, en outre, dans le cas où au moins un dispositif de distillation est constitué par un ou plusieurs plateaux de distillation, au moins un plateau de répartition de liquide (6), traversé par au moins une conduite ou cheminée (dont l'extrêmité supérieure est de préférence saillante et de préférence couverte), sensiblement incliné, situé dans au moins un espace libre se trouvant entre un plateau de distillation et le lit catalytique situé immédiatement au-dessus dudit plateau de distillation, chaque plateau de répartition de liquide étant muni à son extrêmité la plus basse d'au moins un passage libre (qui est, de préférence, une goulotte ou conduite de déversement bordée d'un déversoir).

L'appareil (1) selon la présente invention peut également renfermer au moins un dispositif choisi dans le groupe constitué par :

– au moins un dispositif de distillation situé entre le sommet de l'appareil et le lit catalytique situé le plus haut dans ledit appareil, et,

– au moins un dispositif de distillation situé entre la base de' l'appareil et le lit catalytique situé le plus bas dans ledit appareil.

Le procédé selon l'invention peut être mis en oeuvre dans cet appareil.

L'appareil peut être utilisé, par exemple, pour la préparation du méthyl tertio-butyl éther (MTBE) à partir de méthanol et d'isobutène, pour la préparation du tertio-amyl méthyl éther (TAME) à partir de méthanol et d'isopentène et pour la préparation du MTBE et du TAME à partir de méthanol, d'isobutène et d'isopentène, en présence d'un catalyseur approprié, tel qu'un catalyseur du type résine sulfonée (par exemple, une résine polystyrène-divinylbenzène sulfonée), ces utilisations n'étant pas limitatives.

Exemple (comparatif)

Une charge, renfermant du méthanol et un mélange de butènes et butanes contenant environ 25 % d'isobutène déjà converti à 80 % en MTBE dans un lit de catalyseur du type résine sulfonée, est introduite dans une enceinte renfermant un certain nombre de lits dudit catalyseur et de plateaux de distillation : selon le procédé de l'invention (et en opérant à une pression sensiblement de l'ordre de 10 bars, à une température comprise entre 60 et 135 °C environ et avec un rapport de reflux de l'ordre de 1:1) on peut alors convertir (en MTBE) dans cette enceinte environ 80 % de l'isobutène résiduel en plaçant, en particulier, un plateau de distillation dans chaque espace se trouvant entre deux lits consécutifs dudit catalyseur. On constate que le nombre de lits dudit catalyseur employés dans le procédé selon l'invention est la moitié du nombre de mêmes lits qu'il faut utiliser, pour obtenir le même résultat, dans une colonne catalytique n'utilisant pas l'invention.

## Revendications

1. Procédé de préparation d'un éther alkylique tertiaire (voir figures 1,2 et 3) par réaction d'un alcool aliphatique avec un mélange d'hydrocarbures renfermant au moins une iso-oléfine, dans une zone de réaction-distillation définie par une enceinte (1) de forme sensiblement cylindrique et contenant au moins un catalyseur du type résine sulfonée caractérisé :
   – en ce qu'on introduit la charge de réactifs contenant au moins ledit alcool et au moins ledit mélange d'hydrocarbures dans ladite zone de réaction-distillation qui renferme :
      a) au moins deux lits fixes superposés (2a,2b) et non contigus dudit catalyseur de type résine sulfonée, chacun de ces lits occupant toute la section circulaire de la zone de réaction-distillation, étant disposé sur un support perforé (3a,3b) et comprenant une pluralité de poches de tissu renfermant ledit catalyseur, lesdites poches ménageant entre elles des espaces libres,
      b) au moins une zone de distillation, libre de catalyseur, située dans au moins un espace libre se trouvant entre deux lits consécutifs de catalyseur (2a,2b),
      c) au moins un plateau discontinu de redistribution de liquide (5), libre de catalyseur, situé dans au moins un espace libre se trouvant entre une zone de distillation et le lit de catalyseur situé immédiatement en-dessous de ladite zone de distillation,
   – en ce qu'on maintient des conditions de distillation dans la zone de réaction-distillation, de façon à avoir une phase liquide descendante et une phase vapeur ascendante dans ladite zone,
   – en ce qu'on décharge au sommet (8) de la zone de réaction-distillation une phase vapeur renfermant principalement des hydrocarbures non-convertis, et
   – en ce qu'on soutire à la base (10) de la zone de réaction-distillation une phase liquide renfermant principalement ledit éther alkylique tertiaire.

2. Procédé selon la revendication 1 dans lequel au moins une partie de la phase vapeur déchargée au sommet (8) de la zone de réaction-distillation est condensée, puis renvoyée dans ladite zone sous forme de débit liquide.

3. Procédé selon l'une des revendications 1 et 2 dans lequel au moins une partie de la phase liquide soutirée à la base (10) de la zone de réaction-distillation est vaporisée, puis réintroduite dans ladite zone sous forme de débit vapeur.

4. Procédé selon l'une des revendications 1 à 3 dans lequel l'espace si tué entre le sommet de la zone de réaction-distillation et le lit catalytique situé le plus haut dans ladite zone renferme une zone de distillation, libre de catalyseur.

5. Procédé selon l'une des revendications 1 à 4 dans lequel l'espace si tué entre la base de la zone de réaction-distillation et le lit catalytique situé le plus bas dans ladite zone renferme une zone de distillation, libre de catalyseur.

6. Procédé selon l'une des revendications 1 à 5 dans lequel au moins une zone de distillation est constituée par au moins un plateau discontinu de distillation (4a).

7. Procédé selon la revendication 6 dans lequel la phase liquide arrive, sur chaque plateau de distillation situé immédiatement au-dessous d'un lit catalytique, à l'opposé du déversoir dudit plateau de distillation après être passée préalablement sur un plateau de répartition de liquide (6), libre de catalyseur, traversé par au moins une conduite pour le passage de la phase vapeur, sensiblement incliné, situé dans chaque espace libre se trouvant entre un plateau de distillation et le lit catalytique situé immédiatement au-dessus de ce plateau de distillation, chaque plateau de répartition de liquide étant muni à son extrêmité la plus basse d'au moins un passage libre pour la phase liquide.

8. Procédé selon l'une des revendications 1 à 7 caractérisé en ce qu'on introduit la charge de réactifs contenant au moins ledit alcool et au moins ledit mélange d'hydrocarbures dans la zone de réaction-distillation dans une position (7) telle qu'au moins un lit catalytique se trouve au-dessus de ladite position.

9. Procédé selon l'une des revendications 1 à 8 caractérisé en ce qu'on introduit, en plus de la charge, dudit alcool pris isolément dans la zone de réaction-distillation, au moins en un point différent du point d'introduction de ladite charge et situé au voisinage du sommet de ladite zone.

10. Procédé selon l'une des revendications 1 à 9 dans lequel l'alcool aliphatique est le méthanol et l'iso-oléfine est choisie dans le groupe formé par l'isobutène, l'isopentène et un mélange d'isobutène et d'isopentène.

11. Appareil (1) de forme sensiblement cylindrique (voir figures 1, 2 et 3), comportant au moins une conduite (7) pour l'introduction d'une charge, au moins une conduite (8) située au sommet dudit appareil pour le soutirage d'une phase vapeur, au moins une conduite (9) située au voisinage du sommet dudit appareil pour l'alimentation d'un reflux, au moins une conduite (10) située à la base dudit appareil pour le soutirage d'une phase liquide, au moins une conduite (11) située au voisinage de la base dudit appareil pour l'introduction d'une phase au moins partiellement vaporisée, caractérisé en ce qu'il renferme :

a) au moins deux lits catalytiques fixes, superposés et non contigus (2a,2b), chacun de ces lits étant disposé sur un support perforé (3a,3b) et occupant toute la section circulaire interne de l'appareil,

b) au moins un dispositif de distillation, situé dans au moins un espace libre se trouvant entre deux lits catalytiques consécutifs (2a,2b), et

c) au moins un plateau discontinu de redistribution de liquide (5), situé dans au moins un espace libre se trouvant entre un dispositif de distillation et le lit catalytique situé immédiatement en-dessous dudit dispositif de distillation.

12. Appareil selon la revendication 11 caractérisé en ce qu'il comporte en outre au moins un dispositif choisi dans le groupe constitué par :

– au moins un dispositif de distillation, situé entre le sommet de l'appareil et le lit catalytique situé le plus haut dans ledit appareil, et

– au moins un dispositif de distillation, situé entre la base de l'appareil et le lit catalytique situé le plus bas dans ledit appareil.

13. Appareil selon la revendication 11 ou 12 dans lequel au moins un dispositif de distillation est constitué par au moins un plateau discontinu de distillation.

14. Appareil selon la revendication 13 caractérisé en ce qu'il comporte en outre au moins un plateau de répartition de liquide (6), traversé par au moins une conduite, sensiblement incliné, situé dans au moins un espace libre se trouvant entre un plateau de distillation et le lit catalytique situé immédiatement au-dessus dudit plateau de distillation, chaque plateau de répartition de liquide étant muni à son extrêmité la plus basse d'au moins un passage libre.

**Patentansprüche**

1. Verfahren zur Herstellung eines tertiären Alkyläthers (siehe Figuren 1,2 und 3) durch Reaktion eines aliphatischen Alkohols mit einer Mischung von Kohlenwasserstoffen; die zumindest ein Iso-Olefin enthält, in einer Reaktions-Destillations-Zone, die durch ein im wesentlichen zylinderförmiges Reaktionsgefäß (1) gebildet wird und mindestens einen Katalysator vom Typ eines schwefelhaltigen Harzes enthält, dadurch gekennzeichnet,

– daß die Charge der Reagentien,die mindestens den Alkohol und mindestens die Mischung von Kohlenwasserstoffen enthält, in die Reaktions-Destillations-Zone eingeführt wird, die

a) mindestens zwei feste Betten (2a,2b), die Übereinander aber nicht in unmittelbarer Nachbarschaft angeordnet sind, aufweist, die den Katalysator vom Typ eines schwefelhaltigen Harzes enthalten wobei jedes dieser Betten den gesamten runden Querschnitt der Reaktions-Destillations-Zone einnimmt, auf einem perforierten Träger (3a,3b) angeordnet ist und eine Vielfalt von Gewebetaschen umfaßt, die den Katalysator enthalten, wobei zwischen den Taschen Freiräume gebildet sind,

b) mindestens eine Destillationszone aufweist, die frei vom Katalysator ist und in mindestens einem Freiraum zwischen zwei aufeinanderfolgenden Katalysatorbetten (2a, 2b) angeordnet ist,

c) mindestens eine diskontinuierliche Scheibe (5) zur Wiederverteilung der Flüssigkeit aufweist, die frei vom Katalysator ist und in mindestens einem Freiraum angeordnet ist, der sich zwischen einer Destillationszone und dem Katalysatorbett befindet, das direkt unterhalb dieser Destillationszone angeordnet ist,

– daß die Destillationsbedingungen in der Reaktions-Destillations-Zone so aufrechterhalten werden, daß eine flüssige, absteigende Phase und eine aufsteigende Dampfphase in der Reaktions-Destillations-Zone vorliegen,

– daß am Kopf (8) der Reaktions-Destillations-Zone eine Gasphase entnommen wird, die hauptsächlich nicht umgewandelte Kohlenwasserstoffe enthält, und

– daß am Sumpf (10) der Reaktions-Destillations-Zone eine flüssige Phase entzogen wird, die hauptsächlich den tertiären Alkyläther enthält.

2. Verfahren nach Anspruch 1, bei dem zumindest ein Teil der am Kopf (8) der Reaktions-Destillations-Zone entnommenen Dampfphase kondensiert wird und anschließend in Form von flüssigem Durchfluß wieder in die Reaktions-Destillations-Zone geschickt wird.

3. Verfahren nach einem der Ansprüche 1 oder 2, bei dem zumindest ein Teil der am Sumpf (10) der Reaktions-Destillations-Zone entzogenen, flüssigen Phase verdampft wird und anschließend in Form von Durchflußdampf wieder in die Reaktions-Destillations-Zone wieder eingeführt wird.

4. Verfahren nach einem der Ansprüche 1 bis 3, bei dem der Freiraum, der sich zwischen dem Kopf der Reaktions-Destillations-Zone und dem in dieser Zone am weitesten oben angeordneten,katalytischen Bett befindet, eine Destillationszone enthält, die frei vom Katalysator ist.

5. Verfahren nach einem der Ansprüche 1 bis 4, bei dem der Freiraum, der sich zwischen dem Sumpf der Reaktions-Destillations-Zone und dem in dieser Zone am weitesten unten angeordneten, katalytischen Bett befindet, eine Destillations-Zone enthält, die frei von Katalysator ist.

6. Verfahren nach einem der Ansprüche 1 bis 5, bei dem mindestens eine Destillationszone durch

mindestens eine diskontinuierliche Destillationsscheibe (4a) gebildet ist.

7. Verfahren nach Anspruch 6, bei dem die flüssige Phase auf jeder unmittelbar unterhalb eines katalytischen Bettes angeordneten Destillationsscheibe in größt möglicher Entfernung zur Abflußrinne der Destillationsscheibe ankommt, nachdem sie über eine katalysatorfreie Wiederverteilungsscheibe (6) für die Flüssigkeit geleitet wurde, die zumindest eine Führung für den Durchtritt der Dampfphase aufweist, merklich geneigt ist und jeweils in dem Freiraum angeordnet ist, der sich zwischen einer Destillationscheibe und dem katalytischen Bett befindet, das direkt oberhalb dieser Destillationsscheibe angeordnet ist,.wobei jede Wiederverteilungsscheibe für die Flüssigkeit an ihrem untersten Ende mit mindestens einem freien Durchtritt für die flüssige Phase ausgestattet ist.

8. Verfahren nach einem der Ansprüche 1 bis 7, dadurch gekennzeichnet, daß die Charge der Reagentien,die mindestens den Alkohol und mindestens die Mischung der Kohlenwasserstoffe enthält, an einer Position (7) in die Reaktions-Destillations-Zone eingeführt wird, so daß sich mindestens ein katalytisches Bett oberhalb dieser Position (7) befindet.

9. Verfahren nach einem der Ansprüche 1 bis 8, dadurch gekennzeichnet, daß zusätzlich zu der Charge an mindestens einer Position, die sich von der Einführungsposition der Charge unterscheidet und im Bereich des Kopfes der Reaktions-Destillations-Zone angeordnet ist, isolierter Alkohol vom Typ des in der Charge enthaltenen Alkohols eingeführt wird.

10. Verfahren nach einem der Ansprüche 1 bis 9, bei dem der aliphatische Alkohol Methanol ist und das Iso-Olefin aus der Gruppe gewählt wird, die das Iso-Buten, das Iso-Penten und eine Mischung von Iso-Buten und Iso-Penten bildet.

11. Apparatur (1) mit im wesentlichen zylindrischer Form (siehe Figuren 1, 2 und 3) und mit mindestens einer Führung (7) zum Einführen einer Charge, mit mindestens einer Führung (8) zum Entziehen einer dampfförmigen Phase, die im Bereich des Kopfes der Apparatur angeordnet ist, mit mindestens einer Führung (9) zum Einspeisen eines Rücklaufs, die im Bereich des Kopfes der Apparatur angeordnet ist, mit mindestens einer Führung (10) zum Entziehen einer flüssigen Phase, die im Bereich des Sumpfes der Apparatur angeordnet ist, mit mindestens einer Führung (11) zur Einführung einer zumindest teilweise verdampften Phase, die im Bereich des Sumpfes der Apparatur angeordnet ist, dadurch gekennzeichnet, daß die Apparatur

a) mindestens zwei feste, katalytische Betten (2a,2b) aufweist, die übereinander aber nicht in unmittelbarer Nachbarschaft angeordnet sind, wobei jedes dieser Betten auf einem perforierten Träger (3a,3b) angeordnet ist und den gesamten runden Innenquerschnitt der Apparatur einnimmt,

b) mindestens eine Destillationsvorrichtung aufweist, die in mindestens einem Freiraum angeordnet ist, der sich zwischen zwei aufeinanderfolgenden katalytischen Betten (2a,2b) befindet, und

c) mindestens eine diskontinuierliche Wiederverteilungsscheibe (5) für die Flüssigkeit aufweist, die in mindestens einem Freiraum angeordnet ist, der sich zwischen einer Destillationsvorrichtung und dem unmittelbar unter dieser Destillationsvorrichtung angeordneten katalytischen Bett befindet.

12. Apparatur nach Anspruch 11, dadurch gekennzeichnet, daß sie außerdem mindestens eine der folgenden Vorrichtungen aufweist:
– mindestens eine Destillationsvorrichtung, die zwischen dem Kopf der Apparatur und dem in dieser Apparatur am weitesten oben angeordneten katalytischen Bett angeordnet ist und
– mindestens eine Destillationsvorrichtung, die zwischen dem Sumpf der Apparatur und dem in dieser Apparatur am weitestens unten angeordneten katalytischen Bett angeordnet ist.

13. Apparatur nach einem der Ansprüche 11 oder 12, bei der mindestens eine Destillationsvorrichtung durch mindestens eine diskontinuierliche Destillationsscheibe gebildet ist.

14. Apparatur nach Anspruch 13, dadurch gekennzeichnet, daß sie außerdem mindestens eine mit mindestens einer Durchtrittsführung versehene Wiederverteilungsscheibe (6) aufweist, die merklich geneigt ist und in mindestens einem Freiraum angeordnet ist, der sich zwischen einer Destillationsscheibe und dem unmittelbar über dieser Destillationsscheibe angeordneten katalytischen Bett befindet, wobei jede Wiederverteilungsscheibe an ihrem untersten Ende mit mindestens einem freien Durchtritt ausgestattet ist.

**Claims**

1 - A process for manufacturing a tertiary alkyl (see figures 1, 2 and 3) ether by reacting an aliphatic alcohol with a hydrocarbon mixture containing at least one iso-olefin, in a reaction-distillation zone defined by an enclosure (1) of substantially cylindrical shape, containing at least one catalyst of the sulfonated resin type, characterized by the steps of :
– introducing the reactant charge containing at least said alcohol and at least said hydrocarbon mixture into said reaction-distillation zone, which contains :

a) at least two superposed (2a, 2b) and non-contiguous fixed beds of said catalyst of sulfonated resin type, each of said beds occupying the whole circular section of the reaction-distillation zone being placed on a perforated support member (3a, 3b) and comprising a plurality of fabric pockets containing said catalyst, free spaces being provided between said pockets,

    b) at least one catalyst-free distillation zone, located in at least one free space between two consecutive catalyst beds (2a, 2b),

    c) at least one catalyst-free discontinuous tray (5) for liquid redistribution, located in at least one free space between a distillation zone and the catalyst bed just below said distillation zone,

– maintaining distillation conditions in the reaction-distillation zone, so as to have a descending liquid phase and an ascending vapor phase in said zone,

– discharging from the top (8) of the reaction-distillation zone a vapor phase mainly containing unconverted hydrocarbons, and

– withdrawing from the bottom (10) of the reaction-distillation zone a liquid phase mainly containing said tertiary alkyl ether.

2 - A process according to claim 1, wherein at least a part of the vapor phase discharged at the top (8) of the reaction-distillation zone is condensed and then fed back to said zone as liquid flow.

3 - A process according to one of claims 1 and 2, wherein at least a part of the liquid phase withdrawn from the bottom (10) of the reaction-distillation zone is vaporized and then reintroduced into said zone as vapor flow.

4 - A process according to any of claims 1 to 3, wherein the space between the top of the reaction-distillation zone and the uppermost catalyst bed of said zone contains a catalyst-free distillation zone.

5 - A process according to any of claims 1 to 4, wherein the space between the bottom of the reaction-distillation zone and the lowermost catalyst bed of said zone contains a catalyst-free distillation zone.

6 - A process according to any of claims 1 to 5, wherein at least one distillation zone comprises at least one discontinuous distillation tray (4a).

7 - A process according to claim 6, wherein the liquid phase is supplied to each distillation tray just below a catalyst bed, on the side opposite the overflow of said distillation tray, after previous passage over a catalyst-free liquid distribution tray (6), wherethrough passes at least one duct for the vapor phase, substantially inclined, placed in each free space between a distillation tray and the catalyst bed just above said distillation tray, each liquid distribution tray being provided at its lowermost end with at least one free passage-way for the liquid phase.

8 - A process according to any of claims 1 to 7, characterized in that the reactant charge containing at least said alcohol and at least said hydrocarbon mixture is introduced into the reaction-distillation zone at such a level (7) that at least one catalyst bed is above said position.

9 - A process according to any of claims 1 to 8, characterized by further introducing alcohol of said type, in addition to the charge, separately into the reaction-distillation zone, through at least one port different from that used for introducing said charge, located in the vicinity of the top of said zone.

10 - A process according to any of claims 1 to 9, wherein the aliphatic alcohol is methanol and the iso-olefin is selected from the group formed of isobutene, isopentene and a mixture of isobutene with isopentene.

11 - An apparatus (1) of substantially cylindrical shape (see figures 1, 2, and 3) comprising at least one line (7) for introducing a charge, at least one line (8) at the top of the apparatus for withdrawing a vapor phase, at least one line (9) in the vicinity of the top of said apparatus for a reflux feed, at least one line (10) at the bottom of said apparatus for withdrawing a liquid phase, at least one line (11) in the vicinity of the bottom of said apparatus for introducing an at least partially vaporized phase, characterized in that it contains :

    a) at least two superposed non-contiguous fixed catalyst beds (2a, 2b), each of said beds being placed on a perforated support member (3a, 3b), and occupying the whole circular section of the apparatus,

    b) at least one distillation device, placed in at least one free space between two consecutive catalyst beds (2a, 2b), and

    c) at least one discontinuous liquid redistribution tray (5), placed in at least one free space between a distillation device and the catalyst bed just below said distillation device.

12 - An apparatus according to claim 11, characterized in that it further comprises at least one device selected from the following :

    – at least one distillation device, placed between the top of the apparatus and the uppermost catalyst bed of said apparatus, and

    – at least one distillation device placed between the bottom of the apparatus and the lowermost catalyst bed of said apparatus.

13 - An apparatus according to claim 11 or 12 wherein at least one distillation device consists of at least one discontinuous distillation tray.

14 - An apparatus according to claim 13, characterized in that it further comprises at least one liquid distribution tray (6) traversed by at least one duct and substantially inclined, located in at least one free space between a distillation tray and the catalyst bed just above said distillation tray, each liquid distribution tray being provided at its lowermost end with at least one free passage-way.

FIG.1

FIG.2

FIG.3